# EUROPEAN PATENT APPLICATION

(11) **EP 0 761 160 A1**
(43) Date of publication of application: **12.03.1997**
(21) Application number: 96112786.7
(22) Date of filing: 08.08.1996
(51) Int. Cl.: A61B 5/00, A61N 1/372

(54) **Medical system for patients-at-home**

(30) Priority: 09.08.1995 JP 203369/95
(71) Applicant: Akasaka, Noboru, Tokyo (JP); Kurosawa, Masanori, Saku-shi, Nagano-ken (JP); Akai, Koji, Kamakura-shi, Kanagawa-ken (JP)
(72) Inventor: Akasaka, Noboru, Tokyo (JP); Kurosawa, Masanori, Saku-shi, Nagano-ken (JP); Akai, Koji, Kamakura-shi, Kanagawa-ken (JP)
(74) Representative: Wagner, Karl H., Dipl.-Ing.

(57) **Abstract**

By integrally providing by communication network a local medical organization with a variety of medical data measured on a patient staying at home, the medical system of the present invention makes it possible to make an appropriate diagnosis from a long distance. The system is equipped with an improved emergency reporting unit. The system comprises a plurality of home units (12) set up at each of the patient's home, one or more center units at the local medical organization, and a network interconnecting these units via communication lines (13). The medical data are transmitted from the home units to center units via interface units corresponding to various measurement apparatuses. The system also has an emergency reporting function based on vital signs.

## Description

The present invention relates to a medical system for long-distance or remote care of a patient staying at home and in particular relates to a medical system that includes a communication network between the local medical center and a client's home.

As the ratio of old people in the total population rapidly increases these days, a medical system for remote care of patients-at-home is becoming increasingly strongly desirable. In one prior art, such a system is known wherein only some specific functions as receiving electro-cardiogram data from a patient can be, not integrally, performed. A patient at home can utilize such a prior art system which allows data such as blood pressure and electrocardiogram to be automatically transmitted and which also allows the patient to input personal data such as his/her body weight, for example, via a keyboard at a communication terminal or provide his/her answers to questions posed. Further, a pendant-shaped apparatus is also known by which an emergency notice signal can be transmitted to a medical center by being pulled by a patient him/herself when any emergency occurs.

In order to provide patients-at-home with appropriate and sufficient medical care from a distance, such a medical system as proposed is incorporated with (a) a better emergency alert function, (b) a measurement and communication function for measuring electrocardiogram, blood pressure, pulse count, and pulse wave transmitting these integral measured medical data from a patient's home to a local medical center, (c) a function for interfacing with various kinds of medical equipment that correspond to a plurality of symptoms appearing with patients in obtaining the above medical data, and (d) a diagnosis/instruction function that a real time conversation can be conducted orally and via on-screen input/output means of this system between a physician at the local medical center and each of the patients. The specific functions incorporated in the prior art medical care system are insufficient and thus further improvements are required. These are the reasons why prior art systems are not widely utilized among medical organizations today.

An object of the present invention is to improve on the functions of the prior art medical system described above and to integrate all the necessary functions into a single system, whereby patients-at-home are provided with better medical care; also make such a medical system for patients-at-home more widespread and ultimately try to establish a medical institution by which the lives of patients-at-home will be enhanced as their medical conditions are monitored at a distance in an appropriate manner.

The above and other objectives of the present invention will be accomplished by a system which comprises a network formed by a group of home units each of which is set up at each of a plurality of patients' homes and at least one center unit at a medical institute which functions as a local center for the patients-at-home in the community. The home unit as well as the center unit consists of a computer equipped with a high speed microprocessor (CPU) and an appropriate modem which allows bi-directional communication between each of the home units and the medical center, whereby exchanging information on a real time basis and a physician or a nurse can provide the patient-at-home with an instruction suitable to his/her specific bodily condition based on the medical information acquired via the system.

Also, by using the system according to the present invention, it is possible, depending on the patient's actual requirements to keep a vital sign detector mounted on the patient's body turned-on and to monitor from a distance his/her vital signs on the twenty-four hour basis. Upon detection of any interruption of the vital signs, the CPU within the home unit at the patient's assumes that abnormal functionality of the patient's body has occurred and immediately transmits an alert signal to the center unit. This function makes it possible for the medical professionals at the medical center to immediately respond to such an urgent situation on the patient's life in a more certain manner than the prior art pendant shaped apparatus described above.

In the system of the present invention, a home unit can, collectively rather than specifically, obtain basic medical data such as blood pressure, pulse wave, electrocardiogram, cardiac sound, pulmonary sound, body temperature, which data will be transmitted via communication lines to the center unit at the medical center, where physicians, by monitoring the received data, can provide a proper diagnosis on the distant patient.

Fig. 1 is a block diagram of the medical system for clients-at-home in accordance with the present invention.

Fig. 2 is a more detailed block diagram of a home unit in the medical system for clients-at-home in accordance with the present invention.

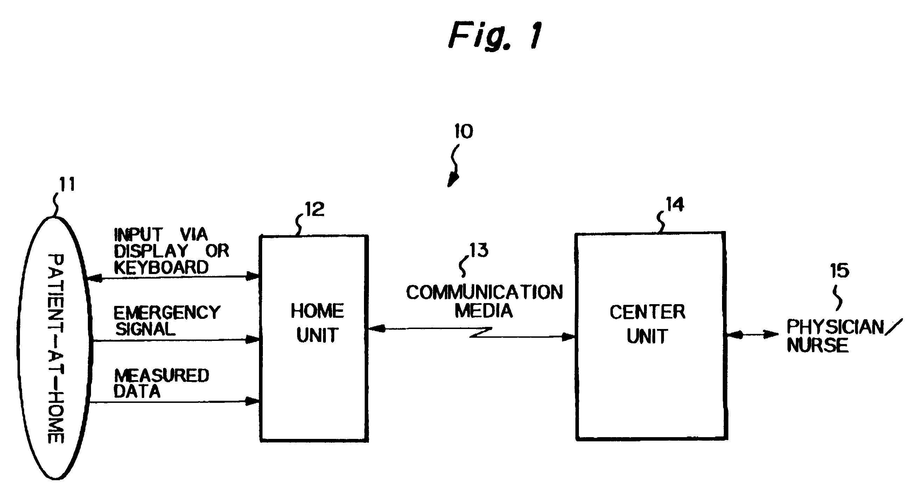

A preferred embodiment of the present invention will now be described by referring to the drawings. Referring to Fig. 1, which is a block diagram of the medical system for clients-at-home in accordance with the present invention, an apparatus set up at a client's home (hereinafter referred to as "home unit") 12 and an apparatus set up at a local medical center (hereinafter referred to as "center unit") 14 are interconnected via a communication line 13 and bi-directional data communication is possible thereover. Each of the home unit 12 and the center unit 14 has a computer capable of providing on-line communication between them. On a display screen of either of the computers at the home unit 12 and the center unit 14, the patient-at-home at the home unit 12 and a physician or nurse 15 at the center unit 14 are able to input necessary data into the computer via an input device such as a touch panel or a keyboard. A diagnosis/instruction via a real-time conversation over the line is also possible. Since a typical patient-at-home 11 of this kind may not be sufficiently computer literate, almost all the functions of the home unit 12, including those for transmitting and receiving, are automated with the data input from the patient-at-home 11 being as minimized as possible.

Communication between the home unit 12 and the center unit 14 may be initiated at either end. For example, an alert notice and measured Bo-medical data can be transmitted from the home unit 12, or medical personnel at the local medical center can themselves check and, if necessary, collect data from the center unit 14.

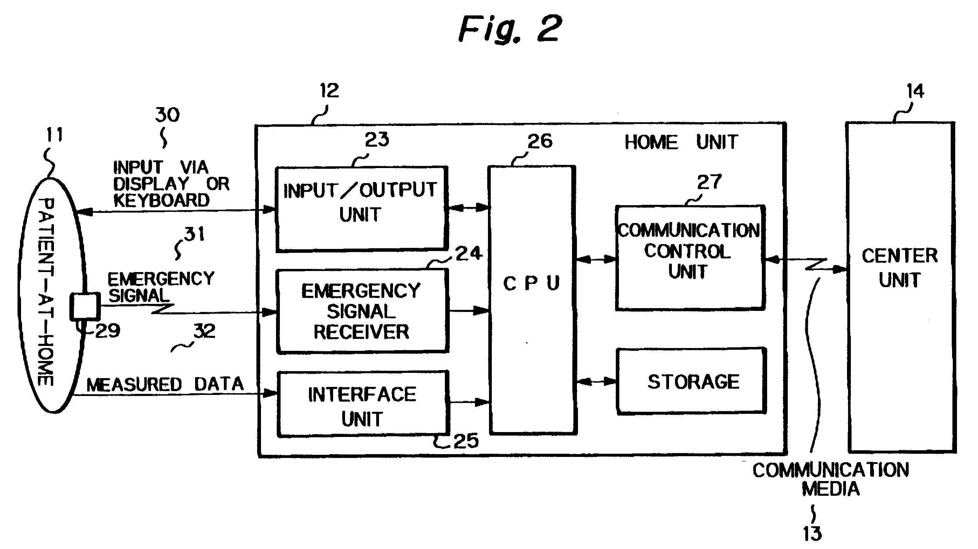

In Fig. 2, the home unit 12 of the system in accordance with the present invention is illustrated in a more detailed manner. The elements shared by Figs. 1 and 2 are identified with the same reference numerals. Detected medical data such as blood pressure, pulse wave, electrocardiogram, cardiac sound, pulmonary sound, body temperature of a patient-at-home are input into the home unit 12 through an input/output unit 23. The patient-at-home 11 is only asked to put on a blood pressure meter or electrodes of an electrocardiograph and turn on the switch, for example, to measure and automatically transmit to the center unit 14 his/her blood pressure or electro-cardiogram. On the display screen at the center unit 14, these transmitted and received data are displayed with the identification number of the patient-at-home 11, which can conveniently be relied upon by a physician or nurse 15 in deciding which measure should be taken if needed.

In the external storage device attached to the computer forming a center unit 14, are stored medical data including biographical and newly measured data of a large number of patients-at-home 11 living in the community which the medical center is in charge of. When a medical professional makes a diagnosis on the basis of the data automatically transmitted from a home unit 12 and received at the center unit 14, he/she can display medical data in one or more desired windows on the display screen by retrieving databases stored in the external storage device and these data are referred to and relied upon. The medical data automatically transmitted from the home unit 12 of a specific patient-at-home 11 to the center unit 14 will be stored in a file specifically assigned to the patient within the database.

Being different from a prior art system, the system of the present invention is not limited to any specific groups of data in obtaining data from a patient-at-home 11 and transmitting them to a medical center but is capable of being tolerant to the kind of data collected and automatically transmitting a variety of data such as blood pressure, pulse wave, electrocardiogram, cardiac sound, pulmonary sound, and body temperature of a patient-at-home 11. Since a diagnosis by a physician can only be made depending upon a plurality of factors collected, it is preferable to collect as many kinds of medical data on the patient as possible. If conventional measurement apparatuses are employed to measure these data, the interface unit 25 gives a good interface between the home unit 12 and a measurement apparatus. It is also possible to use a measurement apparatus dedicated to the home unit 12.

Referring to Fig. 2 again, the interface unit for receiving medical data of the patient 11 receives the data such as blood pressure, pulse wave, electrocardiogram, cardiac sound, pulmonary sound, and body temperature of a patient-at-home 11, converts them into digital form if necessary, and transmits the resultant data in an appropriate format to the center unit 14 via the communication control unit 14. This communication is bi-directional and may be initiated both at the home unit 12 and at center unit 14. The interface unit 25 is configured to automatically adjust the signal level with an input signal so that a variety of medical data can be obtained. The interface unit 25 may be replaced with another interface unit if a medical measurement device requiring such a different unit with a different interface protocol is to be connected.

Even with a prior art system, it was already possible for medical data such as blood pressure, pulse wave, electrocardiogram, and body temperature, which are commonly treated as numerical data, to be measured and transmitted though individually and independently. In contrast to that, the system in accordance with the present invention is further provided with such a function that allows a physician at a local medical center to, remotely or via a communication network, listen to the cardiac and/or pulmonary sound of a patient. Although this is very common routine performed in a face-to-face diagnosis and is regarded as being necessary even in the medical system at home, this function has not been implemented with prior art systems.

Each of the home unit 12 and the center unit 14 may have a microphone/speaker function by which a real time bi-directional conversation will be possible between a patient-at-home 11 and a physician/nurse 15 at a local medical center, wherein participants of the conversation have only to face their own unit 12 or 14 and proceed with the conversation while watching the medical data and/or graphs drawn therefrom displayed on the screen. The physician/nurse 15 may on a real time basis ask questions and give instructions to the patient-at-home. The local medical center often has a plurality of canter units 14 interconnected to one another so that the center is capable of coping with a large number of patients-at-home 11 who live in the community and have entered into a contract with the medical center, whereby each patient-at-home 11 does not have to wait so long before he/she can talk with a physician/nurse 15. Or instead of a real time conversation with a physician/nurse 15, comments on the patient's medical data by a physician/nurse 15 may be transmitted via a center unit 14 to a home unit 12, stored in a storage device such as a RAM, and output as audio data at a moment desired by a patient-at-home 11. By using this function, both the physician/nurse 15 and the patient-at-home 11 may be able to effectively transmit and receive medical instructions without taking too much of the other's time.

With respect to an emergency notice function for reporting to the local medical canter the occurrence of any urgent situation on a patient-at-home's body, the present invention is based on the fail-safe principle and has been significantly improved in terms of reliability over the prior art system. Signals from emergent abnormality detector 29 mounted on the patient-at-home 11 are received by the emergent signal receiver unit 24 of home unit 12, interpreted by the CPU 26, and signals reporting an occurrence of an emergency will be transmitted to a center unit 14. The center unit 14 then recognizes the occurrence of the emergent situation by receiving such signals and immediately notifies the physicians/nurses 15 and the medical center itself of the situation by generating a buzzer sound, for example, from a speaker. At the same time, the center unit 14 transmits an acknowledge signal to the home unit 12 notifying that the medical center has recognized an occurrence of an emergent situation on the patient's body. If the emergent signal from the home unit 12 had been a failure and no emergencies have actually been seen, the home unit 12 then transmits to the center unit 14 such a signal notifying that the prior notice was a failure.

In order to realize the above-mentioned emergency notification function, a small portable apparatus is worn on the patient's body and this apparatus detects the vital signs of the patient which show that the patient is alive. A typical vital sign is the pulse at the patient's wrist. The vital sign detector incorporates a small-sized battery and continues to transmit a constant electrical signal as long as the detector can detect normal vital signs from the patient's body. If the patient stays within a predetermined area, the home unit 12 can receive the electrical signal due to the vital signs. As long as the home unit continues to detect the electrical signal, which confirms that the patient is alive, the home unit 12 does not generate any response. However, once the detection of the signal is discontinued, the home unit 12 will automatically transmit an emergent signal to the center unit 14 without investigating any reason of discontinuance and notifies the medical personnel of an occurrence of an emergent situation. A discontinuance of the electrical signal may be due to other reasons such as electrical or mechanical failures of the detector other than an abnormality in the patient's body, but any discontinuance will be unconditionally reported to the center unit 14 as an occurrence of an emergency as a result of the fail safe principle. On receiving such an emergent signal, the center unit 14 without delay takes necessary measures, namely making a phone call to see if the patient answers the phone, or in other words trying to find out the actual situation.

The prior art pendant-shaped emergent reporting apparatus may not function if the patient is too sick or too weak to pull down the pendant. Thus in a situation where the patient-at-home 11 is suddenly incapacitated or so sick that he/she cannot pull down the pendant-shaped detector to transmit the emergency signal, no signals are generated. The emergency reporting apparatus in accordance with the present invention, however, is configured in such a manner that the home unit 12 always regards the situation as being emergent once the electrical signal discontinues for any reason. Thus reliability is significantly improved compared to the prior art apparatus.

In order for this fail-safe function to operate more efficiently, the emergency notification function can be temporally suspended by setting the home unit 12 to be in an absent mode so that a discontinuance of the electrical signal does not necessarily cause an emergency signal to be generated.

The detected vital signs can be, depending on the condition of the patient, the arterial oxygen saturation (SpO₂) or expired terminal carbon dioxide (ETCO₂). In such a case, the recognition mechanism of the emergency signal will be the same, the only difference being in the detector.

In accordance with the present invention, a variety of physical data can be integrally transmitted to a medical organization without any difficulty, whereby making it possible for a physician/nurse to provide a patient-at-home with remote monitoring and diagnosis. The present system can also provide patients-at-home, the number of which are expected to increase, with better and more efficient diagnoses than prior art systems. Because of the bi-directional communication of this system, patients-at-home may receive real-time diagnoses based on his/her own bodily data via communication media while staying at home.

Further, using the emergency reporting function of this system, the medical organization can be rapidly and with certainty notified of emergent situations of patients-at-home, thereby making appropriate medical treatment at an early stage possible, as well as helping the patients survive such emergencies. Such early treatment also prevents patients from ending up in serious conditions, which may save on extra medical expenses, and with the further result that an efficient allocation of medical resources can be acquired.

While several embodiments of the present invention have bean shown and described, alternate embodiments will be apparent to those skilled in the art and are within the intended scope of the present invention. Therefore, the invention is to be limited only by the following claims.

## Claims

1. A medical system for patients staying at home consisting of home units each of which is set up at each of the homes of patients and comprises a computer with communication capability, center units which are set up at a local medical organization in charge of the patients in the area and each of which comprises a computer with communication capability, and a network inter connecting said home units and said center unit via communication media, said system comprising:
detection means for detecting a plurality of medical data from the patient's body;
means for transmitting the plurality of detected medical data to said center units via interface unit which correspond to said detection means at the home units;
means by which medical personnel at the medical organization may transmit necessary instructions to said home units on the basis of transmitted medical data; and
means for detecting any emergent conditions of the patient and transmitting emergent signals notifying the center units of an occurrence of such an emergent condition.

2. The system recited by claim 1 wherein said means for detecting any emergent conditions of the patient and transmitting emergent signals comprises:
means for detecting vital signs of said patients; and
emergency reporting means for being activated and continuously generating electrical signals as long as said vital signs are being detected and regarding any discontinuance of the electrical signals as being an occurrence of an emergent condition and transmitting emergency signals from said home units to said center unit.

3. The system recited by claim 1 further comprising means for detecting sound waves useful in diagnosis of the patient from the patient's body such as cardiac sound and pulmonary sound and transmitting them to said center units.

4. The system recited by claim 2 wherein said vital signs contain all or some of pulse, SpO₂, and ETCO₂.

5. A medical system for patients staying at home consisting of home units each of which is set up at each of the homes of patients and comprises a computer with communication capability, center units which are set up at a local medical organization in charge of the patients in the area and each of which comprises a computer with communication capability, and a network inter connecting said home units and said center unit via communication media, said system comprising:
detection means for detecting a plurality of medical data from the patient's body.
